# FASCICULE DE BREVET EUROPEEN

(11) **EP 0 854 700 B1**
(45) Date de publication et mention de la délivrance du brevet: **15.05.2002**
(21) Numéro de dépôt: 96933467.1
(22) Date de dépôt: 01.10.1996
(51) Int. Cl.: A61K 7/06, C07K 7/18

(54) **UTILISATION D'ANTAGONISTES DE LA BRADYKININE POUR STIMULER OU INDUIRE LA POUSSE DES CHEVEUX ET/OU STOPPER LEUR CHUTE**
VERWENDUNG VON BRADYKININANTAGONISTEN ZUR INDUZIERUNG ODER STIMULATION DES HAARWACHSTUMS UND/ODER ZUR VERLANGSAMUNG DES HAARAUSFALLS
USE OF BRADYKININ ANTAGONISTS FOR STIMULATING OR INDUCING HAIR GROWTH AND/OR ARRESTING HAIR LOSS

(30) Priorité: 06.10.1995 FR 9511809
(43) Date de publication de la demande: 29.07.1998
(73) Titulaire: L'OREAL, 75008 Paris (FR)
(72) Inventeur: PRUCHE, Francis, 75018 Paris (FR); DURANTON, Albert, 75018 Paris (FR); BOYERA, Nathalie, 75018 Paris (FR); GAUTIER, Brigitte, 91940 Les Ulis (FR)
(74) Mandataire: Tezier Herman, Béatrice
(86) Numéro de dépôt international: FR9601528
(87) Numéro de publication internationale: WO9713493

(56) Documents cités:
- EP-A- 0 334 244
- EP-A- 0 370 453
- EP-A- 0 413 277
- EP-A- 0 455 133
- EP-A- 0 472 220
- EP-A- 0 564 972
- EP-A- 0 578 521
- EP-A- 0 623 350
- WO-A-86/07263
- WO-A-89/01780
- WO-A-89/09230
- WO-A-89/09231
- WO-A-90/03980
- WO-A-91/02746
- WO-A-91/09055
- WO-A-92/17201
- WO-A-93/11789
- WO-A-94/06453
- WO-A-94/11021
- WO-A-94/18835
- WO-A-95/07294

## Description

La présente invention concerne l'utilisation à titre de principe actif, dans un milieu physiologiquement acceptable, dans une composition cosmétique ou pour la préparation d'un médicament, d'une quantité efficace d'au moins un antagoniste de la bradykinine, destinée à induire et/ou stimuler la croissance des cheveux et/ou freiner leur chute.

Chez l'être humain, la croissance des cheveux et leur renouvellement sont principalement déterminés par l'activité des follicules pileux. Leur activité est cyclique et comporte essentiellement trois phases, à savoir la phase anagène, la phase catagène et la phase télogène.

A la phase anagène active ou phase de croissance, qui dure plusieurs années et au cours de laquelle les cheveux s'allongent, succède une phase catagène très courte et transitoire qui dure quelques semaines, puis une phase de repos, appelée phase télogène, qui dure quelques mois.

A la fin de la période de repos, les cheveux tombent et un autre cycle recommence. La chevelure se renouvelle donc en permanence, et sur les 150 000 cheveux environ que comporte une chevelure, à chaque instant, 10% d'entre eux environ sont au repos et seront donc remplacés en quelques mois.

Dans un nombre important de cas, la chute précoce des cheveux survient chez des sujets prédisposés génétiquement et elle atteint notamment les hommes. Il s'agit plus particulièrement de l'alopécie androgénétique ou androgénique ou encore androgéno-génétique.

Cette alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits "terminaux" au stade de duvets. Des zones sont touchées préférentiellement, notamment les golfes temporaux ou frontaux chez l'homme, et chez les femmes, on constate une alopécie diffuse du vertex.

Le terme alopécie recouvre toute une famille d'atteintes du follicule pileux ayant pour conséquence finale la perte définitive partielle ou générale des cheveux. On peut citer, par exemple, l'alopécie androgénétique.

On recherche depuis de nombreuses années, dans l'industrie cosmétique ou pharmaceutique, des substances permettant de supprimer ou de réduire l'alopécie, et notamment d'induire ou de stimuler la croissance des cheveux ou de diminuer leur chute.

Dans cette optique, on a certes déjà proposé un grand nombre de composés actifs très divers, comme par exemple le 2,4-diamino 6-piperidinopyrimidine 3-oxyde ou "Minoxidil" décrit dans US 4 596 812 ou encore ses nombreux dérivés comme ceux décrits par exemple dans les demandes de brevet EP 0353123, EP 0356271, EP 0408442, EP 0522964, EP 0420707, EP 0459890, EP 0519819.

On peut encore citer le 6-amino 1,2-dihydro 1-hydroxy 2-imino 4-pipéridino pyrimidine et ses dérivés, qui sont décrits plus particulièrement dans le brevet US-A-4 139 619.

Il reste, d'une manière générale, qu'il serait intéressant et utile de pouvoir disposer de composés actifs autres que ceux déjà connus, potentiellement plus actifs et/ou moins toxiques.

La bradykinine est un peptide d'origine plasmatique libéré à partir d'un précurseur kininogène par une protéase plasmatique du nom de Kallikreine (EC 3.4.21.24). Ce nanopeptide est un des médiateurs clés de l'inflammation et possède des propriétés mitogénes. Les récepteurs pour cette kinine se divisent en deux principaux sous types, B1 et B2. La bradykinine agit notamment, sur le récepteur B2 et provoque la stimulation de nombreux systèmes de production de seconds messagers dont l'hydrolyse des inositol-phosphates, du métabolisme de l'acide arachidonique, la phosphorylation des résidus tyrosine ainsi que la dépolarisation ou l'hyperpolarisation de la membrane cellulaire.
L'activation de certains récepteurs provoque l'activation de la phospholipase C et donc la production de l'inositol 1,4,5-triphosphate (IP3) et de diacylglycérol (DAG). L'IP3 est connu pour provoquer la libération de calcium à partir des sites de stockages intracellulaires dans les cellules dont le kératinocyte. Le calcium, décrit comme activateur et régulateur de nombreuses enzymes (protéases, phospholipases), joue un rôle important dans la régulation de la différenciation et de la prolifération du kératinocyte.

La bradykinine est impliquée dans un grand nombre de désordres physiopathologiques parmi lesquels : hypotension, contraction des muscles lisses des tractus digestif et respiratoire et de l'utérus, la douleur, la prolifération du tissu conjonctif et la libération de différents médiateurs de l'inflammation : cytokines, leukotriènes et prostaglandines.

A ce jour, à la connaissance de la demanderesse, il n'a pas été envisagé ni même suggéré qu'il existe des récepteurs de la bradykinine dans le follicule pileux, ni que la bradykinine joue un rôle dans les phénomènes aboutissant à la chute et/ou la pousse des cheveux.

D'une façon surprenante et inattendue la demanderesse vient de découvrir que le minoxidil, connu pour ses effets sur la repousse des, cheveux et sur le stokage et/ou le relargage du calcium par la cellule (Matsumoto et coll. Nippon Hifuka Gakkai Zasshi (1993), 103(2), 103-15), bloque l'augmentation de la concentration en calcium du milieu intracellulaire induite par la bradykinine. La demanderesse a également montré qu'il en est de même pour le sulfate de minoxidil, dont l'art antérieur s'accorde à dire qu'il est probablement le dérivé actif du minoxidil sur la repousse des cheveux in vivo.

Ainsi, le minoxidil ou ses dérivés peut agir comme un antagoniste de bradykinine.

Par antagoniste de la bradykinine, on entend tout composé susceptible d'inhiber partiellement, voire totalement, l'effet biologique de la bradykinine, à l'exclusion des composés connus pour avoir un effet sur le stockage et/ou le relargage du calcium dans la cellule, comme le minoxidil et ses dérivés.

Particulièrement, pour qu'une substance soit reconnue comme un antagoniste de la bradykinine elle doit induire une réponse pharmacologique cohérente incluant ou non sa fixation au récepteur de la bradykinine.
Ainsi, entre dans cette définition tout composé qui peut interférer avec les effets de la bradykinine par sa fixation au récepteur de celle-ci (B1 ou B2) et/ou tout composé qui indépendamment de la fixation au(x) récepteur(s) induira par un mécanisme quelconque un effet contraire à celui connu de la bradykinine (par exemple interférant avec la synthèse de la bradykinine).

L'utilisation d'antagoniste de la bradykinine peut alors être l'une des voies efficaces pour lutter contre la chute des cheveux et/ou favoriser leur repousse.

Cette découverte est à la base de la présente invention.

Ainsi, l'invention concerne l'utilisation, pour la préparation d'un médicament, d'une quantité efficace d'au moins un antagoniste de la bradykinine, à l'exception du 2,4-diamino 6-piperidinopyrimidine 3-oxyde et ses dérivés, cet antagoniste ou le médicament étant destiné à induire et/ou stimuler la croissance des cheveux et/ou freiner leur chute.

Selon l'invention, il est possible d'utiliser un seul ou conjointement plusieurs antagonistes de bradykinine. Par exemple, il est possible d'utiliser un antagoniste de libération et/ou de synthèse en association avec un antagoniste réceptoriel B₁ et/ou B₂, par exemple.

Comme il a été spécifié précédement, par antagoniste de la bradykinine on entend, selon l'invention, tout composé susceptible d'inhiber partiellement, voire totalement, l'effet biologique de la bradykinine, à l'exclusion des composés connus pour avoir un effet sur le stokage et/ou le relargage du calcium dans la cellule, comme le minoxidil et ses dérivés.

Parmi les antagonistes de la bradykinine, on préfère utiliser par exemple, des composés inhibant la synthèse et/ou accélérant le catabolisme de la bradykinine, des composés neutralisant la bradykinine, des composés bloquant les récepteurs de la bradykinine tels que ceux qui interférent avec les effets de la bradykinine par leur fixation au récepteur de celle-ci (B1 ou B2), des composés inhibant la synthèse des récepteurs de la bradykinine ou des composés intervenant en modulant le signal transduit par la bradikinine. Ces composés peuvent être d'origine naturelle ou synthétique.

Parmi les antagonistes de la bradykinine, on peut citer plus particulièrement des peptides, synthétiques ou naturels, éventuellement modifiés, comme la D-Arg, [Hyp3, D-Phe7]-bradykinin (NPC567), la [Thi 5, 8, D-Phe7]-bradykinin, la D-Arg, [Hyp3, Thi5,8, D-Phe7]-bradykinin, la N-α-adamantaneacetyl-D-Arg, [Hyp3, Thi5,8, D-Phe7]-bradykinin, la des-Arg9, [Leu8]-bradykinin (tous vendus par la société Sigma) ou encore les composés cités dans les brevets WO 95/08566, WO 95/07294, EP 0623350, EP 0622361, WO 94/11021, EP 0596406, WO 94/06453, WO 94/09001, EP 0578521, EP 0564972, EP 0552106, WO 93/11789, US 5216165, US 5212182, WO 92/17201, EP 0496369, EP 0472220, EP 0455133, WO 91/09055, WO 91/02746, EP 0413277, EP 0370453, EP 0359310, WO 90/03980, WO 89/09231, WO 89/09230, WO 89/01780, EP 0334244, EP 0596406, WO 86/07263 ou la P-guanidobenzoyl, [Hyp3, Thi5, D-Tic7, Oic8]-bradykinin (S 16118) (Feletou M & al., Pharmacol. Exp. Ther., June 1995, 273, 1078-84), la D-Arg, [Hyp3, Thi5, D-Tic7, Oic8]-bradykinin (HOE 140) (Feletou M & al., Eur. J. Pharmacol. 1995, 274, 57-64), la D-Arg, [Hyp3, D-Hype (trans-propyl)7, Oic8]-bradykinin (NPC 17731) ( Herzig M.C.S. and Leeb-Lundberg L.M.F., J. Biol. Chem. 1995, 270, 20591-20598) ou ceux cités dans Bradykinin Antagonists : development and applications (Stewart J.M., Biopolymers, 1995, 37, 143-155), ou encore des molécules chimiques, synthétiques ou naturelles, comme par exemple celles décrites dans Salvino et coll. J. Med. Chem., 1993, 36, 2583-2584.

On peut également utiliser selon l'invention des acides nucléiques antisens ou des ribozymes ayant pour but de sélectivement inhiber la synthèse de la bradykinine. Ces acides nucléiques antisens sont connus de l'homme du métier. Ils peuvent agir de différentes manières sur l'ADN ou sur l'ARN messager codant pour la bradykinine, notamment par blocage de la fixation ou de la progression des ribosomes le long de l'ARN messager, par clivage de l'ARN messager par la RNase H, ou en empêchant le transport de l'ARN messager du noyau vers le cytoplasme ou encore en empêchant la maturation de l'ARN messager.

On peut encore utiliser selon l'invention des anticorps anti-bradykinine ou des récepteurs solubles de la bradykinine, des anticorps anti-récepteurs de la bradykinine ou des antagonistes des récepteurs de la bradykinine.

Préférentiellement, selon l'invention on utilise un composé qui interfére avec les effets de la bradykinine par sa fixation au récepteur de celle-ci (B1 ou B2), préférentiellement au récepteur B2.

Encore plus préférentiellement, on utilise selon l'invention un antagoniste de la bradykinine choisi parmi :
la D-Arg, [Hyp3, D-Phe7]-bradykinin (NPC567),
la [Thi 5, 8, D-Phe7]-bradykinin,
la D-Arg, [Hyp3, Thi5,8, D-Phe7]-bradykinin,
la N-α-adamantaneacetyl-D-Arg, [Hyp3, Thi5,8, D-Phe7]-bradykinin,
la des-Arg9, [Leu8]-bradykinin,
la P-guanidobenzoyl, [Hyp3, Thi5, D-Tic7, Oic8]-bradyklnin (S 16118),
la D-Arg, [Hyp3, Thi5. D-Tic7, Oic8]-bradykinin (HOE 140),
la D-Arg, [Hyp3, D-Hype (trans-propyl)7, Oic8]-bradykinin (NPC 17731)
Le peptide modifié préférentiellement utilisé selon l'invention est la D-Arg, [Hyp3, ThiS, D-Tic7, Oic8]-bradykinin (HOE 140).

La quantité efficace d'antagonistes de la bradykinine à utiliser correspond bien entendu à la quantité nécessaire pour obtenir le résultat désiré. L'homme du métier est donc en mesure d'évaluer cette quantité efficace qui dépend de la nature de l'antagoniste utilisé et de la personne ainsi traitée.

Pour donner un ordre de grandeur, selon l'invention, dans une composition cosmétique, l'antagoniste peut être présent à une concentration comprise entre 10⁻¹² M. et 10⁻³ M. et de préférence comprise entre 10⁻⁹ M. et 10⁻⁴ M.. Dans la préparation d'un médicament, l'inhibiteur peut être présent à une concentration comprise entre 10⁻¹² M. et 1 M. et de préférence comprise entre 10⁻⁶ M. et 10⁻¹ M..

Le médicament selon l'invention peut être administré par voie parentérale, entérale ou encore par voie topique. De préférence, le médicament est administré par voie topique.

Le milieu physiologiquement acceptable dans lequel l'actif est utilisé selon l'invention peut être anhydre ou aqueux. On entend par milieu anhydre, un milieu solvant contenant moins de 1% d'eau. Ce milieu peut être constitué d'un solvant ou d'un mélange de solvants choisi plus particulièrement parmi les alcools inférieurs en C₂-C₄ comme l'alcool éthylique, les alkylèneglycols comme le propylèneglycol, et les alkyléthers d'alkylèneglycols ou de dialkylèneglycols, dont les radicaux alkyle ou alkylène contiennent de 1 à 4 atomes de carbone. On entend par milieu aqueux, un milieu constitué par de l'eau ou un mélange d'eau et d'un autre solvant physiologiquement acceptable, choisi notamment parmi les solvants organiques cités ci-dessus. Dans ce dernier cas, ces autres solvants, lorsqu'ils sont présents, représentent environ 5 à 95% en poids de la composition.

Il est possible que le milieu physiologiquement acceptable puisse contenir d'autres adjuvants habituellement utilisés dans le domaine cosmétique ou pharmaceutique, tels que des agents tensioactifs, des agents épaississants ou gélifiants, des agents cosmétiques, des agents conservateurs, des agents alcalinisants ou acidifiants bien connus dans l'état de la technique, et en quantités suffisantes pour obtenir la forme de présentation désirée, notamment de lotion plus ou moins épaissie, de gel, d'émulsion, ou de crème. L'utilisation peut éventuellement se faire sous une forme pressurisée en aérosol ou vaporisée à partir d'un flacon pompe.

Il est possible aussi d'utiliser en association avec l'actif des composés améliorant encore l'activité sur la repousse et/ou sur le freinage de la chute des cheveux, et ayant déjà été décrits pour cette activité.

Parmi ces derniers composés, on peut plus particulièrement citer à titre non limitatif :
- les esters d'acide nicotinique, dont notamment le nicotinate de tocophérol, le nicotinate de benzyle et les nicotinates d'alkyles en C₁-C₆ comme les nicotinates de méthyle ou d'hexyle ;
- les dérivés de pyrimidine, comme le 2,4-diamino 6-piperidinopyrimidine 3-oxyde ou "Minoxidil" décrit dans US 4 596 812 ou encore ses nombreux dérivés, ou comme le 6-àmino 1,2-dihydro 1-hydroxy 2-imino 4-pipéridinopyrimidine et ses dérivés tels que décrits dans le brevet US 4 139 619 ;
- les agents favorisant la repousse des cheveux comme ceux décrits par la demanderesse dans la demande de brevet européen publiée sous le numéro 0648488 ;
- les agents antibactériens tels que les macrolides, les pyranosides et les tétracyclines, et notamment l'Erythromycine ;
- les agents antagonistes de calcium, comme la Cinnarizine et le Diltiazem ;
- des hormones, telles que l'estriol ou des analogues, ou la thyroxine et ses sels ;
- des agents anti-inflammatoires stéroïdiens, tels que les corticostéroïdes (par exemple : l'hydrocortisone) ;
- des agents antiandrogènes, tels que l'oxendolone, la spironolactone, le diéthylstilbestrol ;
- des antagonistes des 5-α-réductases ;
- des capteurs de radicaux OH, tels que le diméthylsulfoxyde ;

A la liste ci-dessus, d'autres composés peuvent également être rajoutés, à savoir par exemple le Diazoxyde, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'Anthraline, des caroténoides, les acides eicosatétrayénoïque et eicosatriyénoïque ou leurs esters et amides, la vitamine D et ses dérivés.

On peut également envisager que la composition comprenant au moins un antagoniste de la bradykinine soit sous forme liposomée, telle que notamment décrite dans la demande de brevet WO 94/22468 déposée le 13 octobre 1994 par la société Anti Cancer Inc. Ainsi, l'antagoniste encapsulé dans les liposomes peut être délivré sélectivement au niveau du follicule pileux.

La composition cosmétique selon l'invention est à appliquer sur les zones alopéciques du cuir chevelu et des cheveux d'un individu, et est éventuellement laissée en contact plusieurs heures et est éventuellement à rincer. On peut, par exemple, appliquer la composition contenant une quantité efficace d'au moins un un antagoniste de la bradykinine, le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention a également pour objet un procédé de traitement cosmétique des cheveux et/ou du cuir chevelu, caractérisé par le fait qu'il consiste à appliquer sur les cheveux et/ou le cuir chevelu, une composition comprenant une quantité efficace d'au moins un antagoniste de la bradykinine, à laisser celle-ci en contact avec les cheveux et/ou le cuir chevelu, et éventuellement à rincer.

Le procédé de traitement présente les caractéristiques d'un procédé cosmétique dans la mesure où il permet d'améliorer l'esthétique des cheveux en leur donnant une plus grande vigueur et un aspect amélioré.

On va maintenant donner à titre d'illustration des exemples qui ne sauraient limiter en aucune façon la portée de l'invention.

### Exemple 1 :

### Mesure de l'effet du Minoxidil et du Minoxidil sulfate sur l'augmentation de la concentration du calcium intracellulaire induite par la bradykinine :

### a°) Culture de keratinocytes de la gaine externe du follicule pileux humain :

Ce protocole est une variante de celui décrit par YANG et coll., J. Invest. Dermatol., 1993, 101 (5), 652-659.

Une biopsie de scalp de lifting, après décontamination est traitée à la dispase à raison de 2,4 unités / ml pendant 20 heures à 4°C.
La gaine externe du follicule pileux (ORS) est ensuite isolée par dissection, puis traitée à la trypsine à 0.125% pendant 30 minutes à 37°C.
Les cellules ainsi préparées sont ensuite cultivées en milieu Green conditionné pendant 6 jours.
Les cellules sont ensuite déposées sur lamelles de verre de 10 mm de diamètre et cultivées en milieu KGM.

### b°) Mesure de la concentration en calcium intracellulaire :

Ces mesures sont effectuées selon les protocoles classiquement utilisés par l'homme du métier, et que l'on trouve par exemple dans Cellular Calcium : A pratical Approach, ed. J.G. Mc Cormack and P.H. Cobbold ; IRL PRESS (1991).

Les cellules sont incubées pendant 1 heure à 37 °C dans du tampon HEPES 10mM, KCl 5 mM, NaCI 140 mM, Glucose 10 mM, MgCl₂ 1 mM, CaCl₂ 2 mM avec du FURA 2 acetométhylester 5µM et de Pluronic (5 mg / 10 ml).
Après rinçage dans le tampon, les lamelles sont placées dans un support adapté et la lecture s'effectue au spectrofluorimètre PERKIN ELMER LS 50 B, à 37 °C.
Les cellules sont préincubées avec ou sans Minoxidil ou Minoxidil sulfate, pendant 120 secondes au moins avant l'injection de Bradykinine à 1 µM.

### c°) Résultats :

| Produit | c.c.* en µM | % Inhibition (bradykinine 1 µm) |
|---|---|---|
| Minoxidil | 10 | 0% |
| | 50 | 15% |
| | 100 | 25 % |
| | 1000 | 31 % |
| Minoxidil-sulfate | 10 | 0 % |
| | 50 | 31 % |
| | 100 | 54 % |
| | 1000 | 70 % |

| | | |
|---|---|---|
| * c.c. = concentration de produit en µM utilisé dans le test. | | |

Le Minoxidil et le Minoxidil sulfate inhibent l'augmentation de la concentration en calcium intracellulaire induite par la bradykinine, agissant ainsi comme des antagonistes de la bradykinine.

Exemple 2 : Exemples de compositions à appliquer sur les cheveux et/ou le cuir chevelu. Ces compositions peuvent se préparer par simple mélange.

| Composition 1 : Spray : | |
|---|---|
| D-Arg, [Hyp3, Thi5, D-Tic7,Oic8]-bradykinirte (HOE 140) | 5.10⁻⁶ g |
| Minoxidil | 0,5 g |
| Ethanol à 95° | 55,1 g |
| Propylène glycol | 22,8 g |
| Parfum | qs |
| Eau déminéralisée qsp | 100 g |

| Composition 2 : Lotion quotidienne : | |
|---|---|
| D-Arg, [Hyp3, D-Phe7]-bradykinin (NPC567) | 12,5 10⁻⁶ g |
| 2,4 diaminopyrimidine-3-oxyde | 0,75 g |
| Ethanol à 95° | 30 g |
| Parfum | qs |
| Colorants | qs |
| Eau déminéralisée qsp | 100 g |

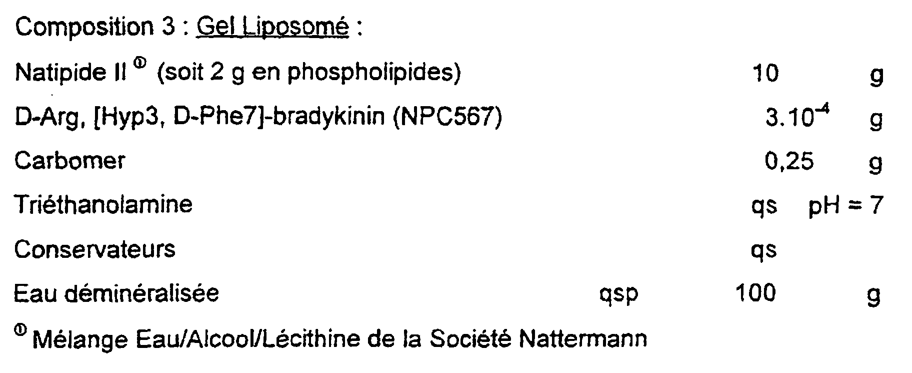

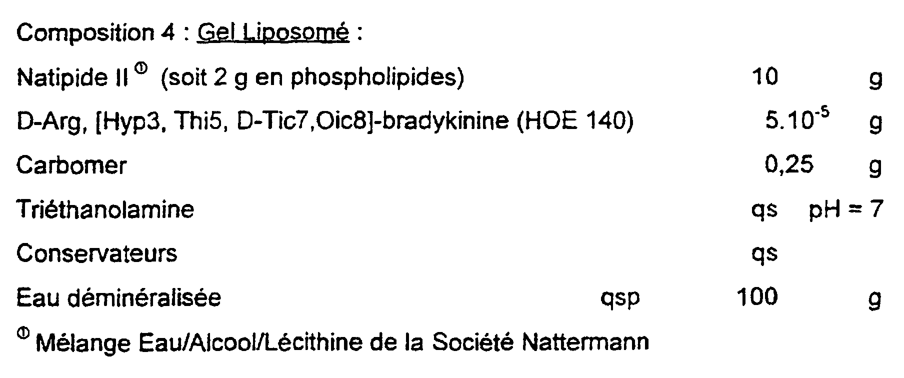

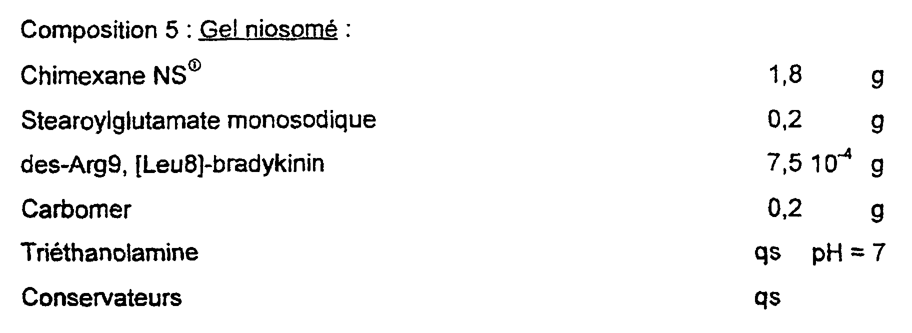

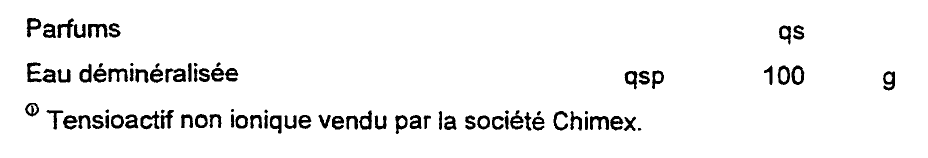

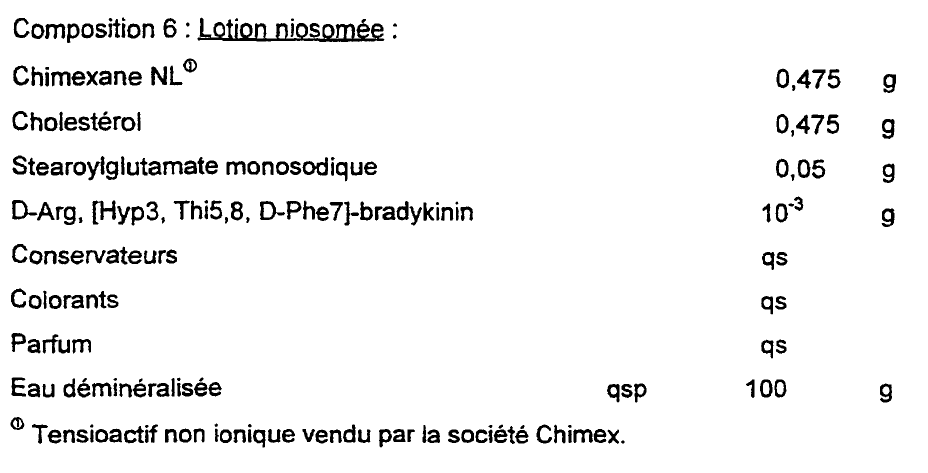

| Composition 7 : Crème de soin : émulsion H/E | |
|---|---|
| Alcool cétylstéarylique/Alcool cétylstéarylique oxyéthylénée à 33 moles d'oxyéthylène (80/20) | 5 g |
| Monostéarate de glycérol | 1,5 g |
| Alcool cétylique | 0,75 g |
| Huile de vaseline | 10 g |
| Polydimethylsiloxane | 0,75 g |
| Glycérine | 4 g |
| Conservateurs | qs |
| D-Arg, [Hyp3, Thi5, D-Tic7,Oic8]-bradykinine (HOE 140) | 5.10⁻³ g |
| Eau déminéralisée qsp | 100 g |

## Revendications

1. Utilisation, pour la préparation d'un médicament, d'une quantité efficace d'au moins un antagoniste de la bradykinine, à l'exception du 2,4-diamino 6-piperidinopyrimidine 3-oxyde et ses dérivés, cet antagoniste ou le médicament étant destiné à induire et/ou stimuler la croissance des cheveux et/ou freiner leur chute.

2. Utilisation selon la revendication précédente, **caractérisée par le fait que** l'antagoniste de la bradykinine est choisi parmi les composés inhibant la synthèse et/ou accélérant le catabolisme de la bradykinine, les composés neutralisant la bradykinine, les composés bloquant les récepteurs de la bradykinine tels que ceux qui interférent avec les effets de la bradykinine par leur fixation au récepteur de celle-ci (B1 ou B2), les composés inhibant la synthèse des récepteurs de la bradykinine ou les composés intervenant en modulant le signal transduit par la bradykinin.

3. Utilisation selon l'une quelconque des revendications précédentes, **caractérisée par le fait que** l'antagoniste de la bradykinine est choisi parmi des peptides, synthétiques ou naturels, éventuellement modifiés, des molécules chimiques, synthétiques ou naturelles, des acides nucléiques antisens, des ribozymes, des anticorps anti-bradykinines, des récepteurs solubles de la bradykinine, des anticorps anti-récepteurs de la bradykinine ou des antagonistes des récepteurs de la bradykinine.

4. Utilisation selon l'une quelconque des revendications précédentes, **caractérisée par le fait que** l'antagoniste de la bradykinine est choisi parmi les composés qui interférent avec les effets de la bradykinine par leur fixation au récepteur de celle-ci (B1 ou B2) et préférentiellement au récepteur B2.

5. Utilisation selon l'une quelconque des revendications précédentes, **caractérisée par le fait que** l'antagoniste de la bradykinine est choisi parmi
la D-Arg, [Hyp3, D-Phe7]-bradykinine (NPC567),
la [Thi 5, 8, D-Phe7]-bradykinine,
la D-Arg, [Hyp3, Thi5,8, D-Phe7]-bradykinine,
la N-α-adamantaneacetyl-D-Arg, [Hyp3, Thi5,8, D-Phe7]-bradykinine,
la des-Arg9, [Leu8]-bradykinine,
la P-guanidobenzoyl, [Hyp3,Thi5,D-Tic7, Oic8]-bradykinine (S 16118),
la D-Arg, [Hyp3, Thi5, D-Tic7, Oic8]-bradykinine(HOE 140),
la D-Arg, [Hyp3, D-Hype (trans-propyl)7, Oic8]-bradykinine (NPC 17731).

6. Utilisation selon la revendication précédente, **caractérisée par le fait que** l'antagoniste de la bradykinine est la D-Arg, [Hyp3, Thi5, D-Tic7, Oic8]-bradykinine (HOE 140).

## Patentansprüche

1. Verwendung einer wirksamen Menge mindestens eines Bradykinin-Antagonisten, wobei das 2,4-Diamino-6-piperidinopyrimidin-3-oxid und seine Derivate ausgenommen sind, zur Herstellung von Arzneimitteln, wobei der Antagonist oder das Arzneimittel zur Induzierung und/oder Stimulation des Haarwachstums und/oder zur Verlangsamung des Haarausfalls dienen sollen.

2. Verwendung nach dem vorhergehenden Anspruch, **dadurch gekennzeichnet, daß** der Antagonist von Bradykinin unter den Verbindungen, die die Synthese von Bradykinin hemmen und/oder den Katabolismus von Bradykinin beschleunigen, den Verbindungen, die Bradykinin neutralisieren, den Verbindungen, die die Rezeptoren von Bradykinin blockieren, wie beispielsweise die Verbindungen, die die Wirkungen des Bradykinins stören, indem sie sich an den Rezeptor des Bradykinins binden (B1 oder B2), den Verbindungen, die die Synthese der Rezeptoren von Bradykinin hemmen, und den Verbindungen, die dadurch eingreifen, indem sie das durch Bradykinin vermittelte Signal modulieren, ausgewählt ist.

3. Verwendung nach einem der vorhergehenden Ansprüche, **dadurch gekennzeichnet, daß** der Antagonist von Bradykinin unter synthetischen und natürlichen, gegebenenfalls modifizierten Peptiden, synthetischen und natürlichen chemischen Molekülen, Antisense-Nucleinsäuren, Ribozymen, anti-Bradykinin-Antikörpern, löslichen Rezeptoren von Bradykinin, Antikörpern gegen die Rezeptoren von Bradykinin und Antagonisten der Rezeptoren von Bradykinin ausgewählt ist.

4. Verwendung nach einem der vorhergehenden Ansprüche, **dadurch gekennzeichnet, daß** der Antagonist von Bradykinin unter den Verbindungen ausgewählt ist, die die Wirkungen des Bradykinins stören, indem sie sich an den Rezeptor des Bradykinins (B1 oder B2) und vorzugsweise an den B2-Rezeptor binden.

5. Verwendung nach einem der vorhergehenden Ansprüche, **dadurch gekennzeichnet, daß** der Antagonist von Bradykinin ausgewählt ist unter:
D-Arg, [Hyp3, D-Phe7]-bradykinin (NPC576),
[Thi 5,8, D-Phe7]-bradykinin,
D-Arg,[Hyp3, Thi5,8, D-Phe7]-bradykinin,
N-α-Adamantanacetyl-D-Arg, [Hyp3, Thi5,8, D-Phe7]-bradykinin,
des-Arg9, [Leu8]-bradykinin,
P-Guanidobenzoyl,[Hyp3, Thi5, D-Tic7, Oic8]-bradykinin (S 16118),
D-Arg,[Hyp3,Thi5,D-Tic7, Oic8]-bradykinin (HOE 140), und
D-Arg,[Hyp3, D-Hype (trans-propyl)7, Oic8]-bradykinin (NPC 17731)

6. Verwendung nach dem vorhergehenden Anspruch, **dadurch gekennzeichnet, daß** der Antagonist von Bradykinin das D-Arg,[Hyp3, Thi5, D-Tic7, Oic8]-bradykinin (HOE 140) ist.

## Claims

1. Use, for the preparation of a medicinal product, of an effective amount of at least one bradykinin antagonist with the exception of 2,4-diamino-6-piperidinopyrimidine 3-oxide and derivatives thereof, this antagonist or the medicinal product being intended to induce and/or stimulate hair growth and/or slow down hair loss.

2. Use according to the preceding claim, **characterized in that** the bradykinin antagonist is chosen from compounds which inhibit the synthesis and/or accelerate the catabolism of bradykinin, bradykinin neutralizers, bradykinin receptor blockers such as those which interfere with the effects of bradykinin by binding to its receptor (B1 or B2), compounds which inhibit the synthesis of bradykinin receptors or compounds involved in modulating the signal transduced by bradykinin.

3. Use according to either of the preceding claims, **characterized in that** the bradykinin antagonist is chosen from optionally modified, natural or synthetic peptides, natural or synthetic chemical molecules, antisense nucleic acids, ribozymes, anti-bradykinin antibodies, soluble bradykinin receptors, anti-bradykinin-receptor antibodies or bradykinin receptor antagonists.

4. Use according to any one of the preceding claims, **characterized in that** the bradykinin antagonist is chosen from compounds which interfere with the effects of bradykinin by binding to its receptor (B1 or B2) and preferably to the B2 receptor.

5. Use according to any one of the preceding claims, **characterized in that** the bradykinin antagonist is chosen from
D-Arg, [Hyp3, D-Phe7]bradykinin (NPC567),
[Thi5,8, D-Phe7]bradykinin,
D-Arg, [Hyp3, Thi5,8, D-Phe7]bradykinin,
N-α-adamantaneacetyl-D-Arg, [Hyp3, Thi5,8, D-Phe7]-bradykinin,
des-Arg9, [Leu8]bradykinin,
P-guanidobenzoyl, [Hyp3, Thi5, D-Tic7, Oic8]bradykinin (S 16118),
D-Arg, [Hyp3, Thi5, D-Tic7, Oic8]bradykinin (HOE 140),
D-Arg, [Hyp3, D-Hype (transpropyl)7, Oic8] bradykinin (NPC 17731).

6. Use according to the preceding claim, **characterized in that** the bradykinin antagonist is D-Arg, [Hyp3, Thi5, D-Tic7, Oic8]bradykinin (HOE 140).
